(19) Europäisches Patentamt
European Patent Office
Office européen des brevets

(11) Publication number : 0 387 808 B1

# (12) EUROPEAN PATENT SPECIFICATION

(45) Date of publication of patent specification : 12.05.93 Bulletin 93/19

(51) Int. Cl.$^5$ : **A61K 31/59**, A61K 47/32, A61K 47/38, A61K 9/20

(21) Application number : 90104750.6

(22) Date of filing : 13.03.90

## (54) Compositions for the preparation of dosage-form active vitamins D3 and process for preparing stable dosage-form active vitamins D3 by using the same.

(30) Priority : 13.03.89 JP 60554/89

(43) Date of publication of application : 19.09.90 Bulletin 90/38

(45) Publication of the grant of the patent : 12.05.93 Bulletin 93/19

(84) Designated Contracting States : BE CH DE ES FR GB IT LI NL SE

(56) References cited : EP-A- 0 215 596

(73) Proprietor : SS PHARMACEUTICAL CO., LTD.
2-12-4 Nihonbashihama-cho
Chuo-ku, Tokyo 103 (JP)

(72) Inventor : Moroi, Masami
44-12, Oowada
Yachiyo-shi, Chiba-ken 276 (JP)
Inventor : Yokoyama, Toshio
7-19-5-109 Machiya
Arakawa-ku, Tokyo 116 (JP)
Inventor : Iwasa, Akira
886-16 Shikawatashi
Yotsukaido-shi, Chiba-ken 284 (JP)

(74) Representative : Wächtershäuser, Günter, Dr.
Tal 29
W-8000 München 2 (DE)

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

### BACKGROUND OF THE INVENTION

1) Field of the Invention:

The present invention relates to compositions for the preparation of dosage-form active vitamins $D_3$ in which the active vitamins $D_3$ have superb stability.

2) Description of the Related Art:

Active vitamins $D_3$ such as 1α-hydroxyvitamin $D_3$ and 1α,25-dihydroxyvitamin $D_3$ are known as excellent therapeutic drugs for e.g. rickets and osteomalacia. These active vitamins $D_3$ are however very unstable substances, so that they undergo decomposition even at room temperature and cannot be stored for long time even when prepared into dosage forms.

As their stabilization method, it has heretofore been known, for example, to form each active vitamin $D_3$ into a composition by dissolving it in triglyceride (Japanese Patent Application Laid-Open No. 130,905/1977), to add an amino acid as a stabilizer (Japanese Patent Application Laid-Open No. 17/1987, EP-A-191 489), to form it into a composition by dissolving the same in a propylene glycol fatty acid ester (Japanese Patent Application Laid-Open No. 54323/1988) or to form it into a lyophilised product (EP-A-215 596)..

These methods are however not fully satisfactory. There has hence been a desire for the development of a still better method for the stabilization of active vitamins $D_3$.

### SUMMARY OF THE INVENTION

With the foregoing circumstances in view, the present inventors have carried out an extensive investigation. As a result, it has been found that polyvinylacetal diethylaminoacetate and hydroxypropylcellulose have excellent stabilization effects to active vitamins $D_3$, leading to the completion of the present invention.

In one aspect of the present invention, there is thus provided a composition for the preparation of a dosage-form active vitamin $D_3$, which comprises an active vitamin $D_3$ and a stabilizer selected from polyvinylacetal diethylaminoacetate and hydroxypropylcellulose.

In another aspect of the present invention, there is also provided a method for stabilizing an active vitamin $D_3$, which comprises adding to the active vitamin $D_3$ a stabilizer selected from polyvinylacetal diethylaminoacetate and hydroxypropylcellulose.

In a further aspect of the present invention, there is also provided a process for preparing a stable dosage-form active vitamin $D_3$, which comprises adding to an active vitamin $D_3$ a stabilizer selected from polyvinylacetal diethylaminoacetate and hydroxypropylcellulose and then adding a pharmaceutically-acceptable carrier to the resultant mixture.

According to the present invention, compositions containing an active vitamin $D_3$ with considerably improved stability can be obtained. These compositions are extremely useful for dosage-form preparations.

### DETAILED DESCRIPTION OF THE INVENTION AND THE PREFERRED EMBODIMENTS

Exemplary active vitamin $D_3$ useful in the practice of the present invention include those having a hydroxyl group on 1α-position such as 1α-hydroxyvitamin $D_3$ (1α-OH-$D_3$), 1α,24-dihydroxyvitamin $D_3$ [1α,24-$(OH)_2$-$D_3$], 1α,25-dihydroxyvitamin $D_3$ [1α,25-$(OH)_2$-$D_3$] and 1α,24,25-trihydroxyvitamin $D_3$ [1α,24,25-$(OH)_3$-$D_3$]; and those having no hydroxyl group on 1α-position such as 24-hydroxyvitamin $D_3$ (24-OH-$D_3$) and 25-hydroxyvitamin $D_3$ (25-OH-$D_3$).

Of the stabilizers usable in the present invention, polyvinylacetal diethylaminoacetate means a polyacetal formed by reacting polyvinyl alcohol and acetaldehyde with the removal of $H_2O$ molecules and then causing diethylaminoacetic acid to form ester bonds with some of the remaining hydroxyl groups. It is represented by the following for-

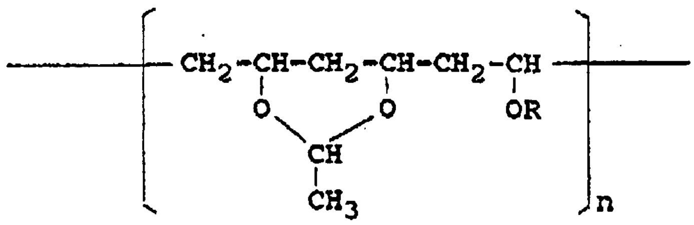

wherein R means H or

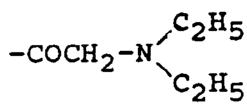

.

In the present invention, polyvinylacetal diethylaminoacetate and hydroxypropylcellulose can be used either singly or in combination. They may be incorporated in a total proportion of 1-100,000 times by weight the active vitamin $D_3$, with 10-50,000 times by weight being particularly preferred.

The composition of the present invention for the preparation of a dosage-form active vitamin $D_3$ can be produced by blending an active vitamin $D_3$ and polyvinylacetal diethylaminoacetate and/or hydroxypropylcellulose into a uniform mixture. As a preferred production method, the following method may be mentioned. Namely, the composition of the present invention for the preparation of a dosage-form active vitamin $D_3$ can be obtained by dissolving an active vitamin $D_3$ in a solvent capable of dissolving the same, for example, ethanol, propanol or isopropanol, adding polyvinylacetal diethylaminoacetate and/or hydroxypropylcellulose to the solution, thoroughly stirring the resultant mixture into a solution and then distilling off the solvent.

To obtain an active-vitamin-$D_3$-containing drug preparation by using the composition obtained as described above, it is only necessary to combine the composition with a pharmaceutically-acceptable known carrier and/or a pharmaceutically-effective known ingredient and then form them into a dosage form. For example, the combination with pharmaceutically-acceptable carriers known to date makes it possible to provide active vitamin $D_3$ preparations such as tablets, granules and capsules. On the other hand, the combination with pharmaceutically-effective ingredients such as vitamins of other kinds leads to the formation of combined drugs such as vitamin complexes.

Since the stability of the active vitamin $D_3$ in the composition of the present invention has been considerably enhanced owing to the action of the associated stabilizer, the composition can be prepared into dosage forms by conventional dosage preparation methods. Further, the active vitamin $D_3$ can be stored stably after the preparation into the dosage forms.

[Examples]

The present invention will next be described in further detail by the following examples.

Example 1

Two milligrams of 1α-OH-$D_3$ were dissolved in 50 mℓ of ethanol so that a solution was prepared. After 2 g of polyvinylacetal diethylaminoacetate ("AEA SANKYO", trade name; product of Sankyo Co., Ltd.) were added to the solution and dissolved therein, ethanol was distilled off under reduced pressure. The residue was dried to obtain 1,900 mg of a composition. The content of 1α-OH-$D_3$ in the composition was 0.1 wt.%.

Example 2

In a similar manner to Example 1 was produced a composition consisting of a mixture of 2 mg of 1α-OH-$D_3$ and 2 g of hydroxypropylcellulose ("HPC-L", trade name; product of Nippon Soda Co., Ltd.).

Example 3

In a similar manner to Example 1 was produced a composition consisting of a mixture of 2 mg of 1α-OH-$D_3$, 1 g of polyvinylacetal diethylaminoacetate ("AEA SANKYO", trade name; product of Sankyo Co., Ltd.) and

1 g of hydroxypropylcellulose ("HPC-L", trade name; product of Nippon Soda Co., Ltd.).

Test 1:

The compositions obtained in Examples 1-3 respectively were stored at 50°C and the percent remainders of 1α-OH-$D_3$ were investigated 2 weeks and 4 weeks later. Employed as a control was a composition which had been obtained by adding 1 g of lactose to an ethanol solution of 1 mg of 1α-OH-$D_3$ and then distilling off ethanol under reduced pressure.

Time-dependent changes of the percent remainders of 1α-OH-$D_3$ in the compositions of the present invention and the control are shown in Table 1. As is clearly envisaged from Table 1, 1α-OH-$D_3$ in each composition of the present invention is far superior in stability compared with that in the control.

Table 1

| Sample | Initial amount, % | Percent remainder after 2 weeks | Percent remainder after 4 weeks |
|---|---|---|---|
| Example 1 | 100.0 | 98.8 | 97.5 |
| Example 2 | 100.0 | 98.4 | 96.2 |
| Example 3 | 100.0 | 98.0 | 95.7 |
| Control | 100.0 | 33.6 | 0.0 |

Example 4

The composition of the present invention, which had been obtained in Example 1, was mixed with other ingredients in accordance with the below-described formula. The resultant mixture was then pressed into vitamin $D_3$ tablets by "KIKUSUI Rotary Tablet Machine" (trade name). The tablets had a diameter of 6 mm, a thickness of about 2 mm and a weight of 85 mg. They contained about 1 μg of 1α-OH-$D_3$ per tablet.

(Formula)

| | |
|---|---|
| Composition of Example 1 | 1.00 g |
| Lactose | 78.05 g |
| Crystalline cellulose | 4.25 g |
| Stearic acid | 1.70 g |

Example 5:

The composition of the present invention, which had been obtained in Example 2, was mixed with other ingredients in accordance with the below-described formula. The resultant mixture was then granulated by an extruder ("W-pelleter Model EXR-60", trade name; manufactured by Fuji Paudal Co., Ltd.). The granules were dried at 50°C and then packaged in stick-like envelopes, whereby a granular vitamin $D_3$ preparation was obtained in the envelopes, each containing 1 g of the granules. The granular preparation contained about 1 μg of 1α-OH-$D_3$ per envelope.

(Formula)

| | |
|---|---|
| Composition of Example 2 | 1.00 g |
| Purified sucrose | 969.00 g |
| Polyvinylpyrrolidone K-90 | 30.00 g |
| Perfume | trace |

Example 6:

The composition of the present invention, which had been obtained in Example 3, was mixed with other ingredients in accordance with the below-described formula. The resultant mixture was then granulated by the extruder ("W-pelleter Model EXR-60", trade name; manufactured by Fuji Paudal Co., Ltd.). The granules were rounded into spherical granules in "Marumerizer Model Q-230" (trade name; manufactured by Fuji Paudal Co., Ltd.), dried at 50°C and then filled in No. 1 capsules at a rate of 350 mg per capsule, whereby capsules were obtained. Those capsules contained about 1 μg of 1α-OH-$D_3$ per capsule.

(Formula)

| | |
|---|---|
| Composition of Example 3 | 1.00 g |
| Purified sucrose | 229.00 g |
| Corn starch | 100.00 g |
| Carboxymethylcellulose | 20.00 g |

## Claims

**Claims for the following Contracting States : BE, CH, DE, FR, GB, IT, LI, NL, SE**

1. A composition for the preparation of a dosage-form active vitamin $D_3$, which comprises an active vitamin $D_3$ and a stabilizer selected from polyvinylacetal diethylaminoacetate and hydroxypropylcellulose.

2. The composition of claim 1, wherein the proportion of the stabilizer is 1-100,000 times by weight the proportion of the active vitamin $D_3$.

3. A method for stabilizing an active vitamin $D_3$, which comprises adding to the active vitamin $D_3$ a stabilizer selected from polyvinylacetal diethylaminoacetate and hydroxypropylcellulose.

4. A process for preparing a stable dosage-form active vitamin $D_3$, which comprises adding to an active vitamin $D_3$ a stabilizer selected from polyvinylacetal diethylaminoacetate and hydroxypropylcellulose and then adding a pharmaceutically-acceptable carrier to the resultant mixture.

5. The process of claim 4, further comprising adding another pharmaceutically-effective ingredient.

**Claims for the following Contracting State : ES**

1. A method for stabilizing an active vitamin $D_3$, which comprises adding to the active vitamin $D_3$ a stabilizer selected from polyvinylacetal diethylaminoacetate and hydroxypropylcellulose.

2. A process for preparing a stable dosage-form active vitamin $D_3$, which comprises adding to an active vitamin $D_3$ a stabilizer selected from polyvinylacetal diethylaminoacetate and hydroxypropylcellulose and then adding a pharmaceutically-acceptable carrier to the resultant mixture.

3. The process of claim 2, further comprising adding another pharmaceutically-effective ingredient.

4. The process of claim 2, wherein the proportion of the stabilizer is 1-100,000 times by weight the proportion of the active vitamin $D_3$.

## Patentansprüche

### Patentansprüche für folgende Vertragsstaaten : BE, CH, DE, FR, GB, IT, LI, NL, SE

1. Zusammensetzung zur Zubereitung einer Dosierungsform von aktivem $D_3$-Vitamin, die ein aktives $D_3$-Vitamin und einen Stabilisator enthält, ausgewählt aus Polyvinylacetaldiethylaminoacetat und Hydroxypropylcellulose.

2. Zusammensetzung nach Anspruch 1, worin der Anteil des Stabilisators das 1-100.000-fache des Gewichts des aktiven Vitamins $D_3$ ausmacht.

3. Verfahren zum Stabilisieren eines aktiven $D_3$-Vitamins, bei dem zu dem aktiven $D_3$-Vitamin ein Stabilisator hinzugegeben wird, der ausgewählt ist aus Polyvinylacetaldiethylaminoacetat und Hydroxypropylcellulose.

4. Verfahren zum Herstellen eines stabilen aktiven $D_3$-Vitamins in Dosierungsform, bei dem zu einem aktiven $D_3$-Vitamin ein Stabilisator hinzugegeben wird, der ausgewählt ist aus Polyvinylacetaldiethylaminoacetat und Hydroxypropylcellulose mit nachfolgendem Hinzugeben eines pharmazeutisch akzeptablen Trägers zu der resultierenden Mischung.

5. Das Verfahren nach Anspruch 4, bei dem noch ein anderer pharmazeutisch wirksamer Bestandteil hinzugegeben wird.

### Patentansprüche für folgenden Vertragsstaat : ES

1. Ein Verfahren zum Stabilisieren eines aktiven $D_3$-Vitamins, umfassend die Zugabe eines Stabilisators, ausgewählt aus Polyvinylacetal-diethylaminoacetat und Hydroxypropylcellulose, zu dem aktiven $D_3$-Vitamin.

2. Ein Verfahren zum Herstellen einer stabilen Dosierungsform von aktivem Vitamin $D_3$, umfassend die Zugabe eines Stabilisators, ausgewählt aus Polyvinylacetal-diethylaminoacetat und Hydroxypropylcellulose, zu einem aktiven $D_3$-Vitamin und nachfolgender Zugabe eines pharmazeutisch akzeptablen Trägers zu der resultierenden Mischung.

3. Das Verfahren nach Anspruch 2, weiter umfassend die Zugabe eines anderen pharmazeutisch wirksamen Bestandteils.

4. Das Verfahren nach Anspruch 2, worin der Anteil des Stabilisators das 1- bis 100.000-fache, bezogen auf das Gewicht, des Anteils des aktiven $D_3$-Vitamins beträgt.

## Revendications

### Revendications pour les Etats contractants suivants : BE, CH, DE, FR, GB, IT, LI, NL, SE

1. Composition pour la préparation d'une vitamine $D_3$ active sous une forme pour l'administration, comprenant une vitamine $D_3$ active et un stabilisant choisi parmi le polyvinylacétal diéthylaminoacétate et l'hydroxypropylcellulose.

2. Composition selon la revendication 1, dans laquelle la proportion de stabilisant est de 1 à 100.000 fois le poids de la teneur en vitamine $D_3$ active.

3. Procédé pour stabiliser une vitamine $D_3$ active comprenant l'addition à la vitamine $D_3$ active d'un stabilisant choisi parmi le polyvinylacétal diéthylaminoacétate et l'hydroxypropylcellulose.

4. Procédé de préparation d'une vitamine $D_3$ active et stable sous une forme pour l'administration, comprenant l'addition à une vitamine $D_3$ active d'un stabilisant choisi parmi le polyvinylacétal diéthylaminoacétate et l'hydroxypropylcellulose, puis !'addition d'un support pharmaceutiquement acceptable au mélange obtenu.

5. Procédé selon la revendication 4, comprenant en outre l'addition d'un autre ingrédient pharmaceutiquement efficace.

**Revendications pour l'Etat contractant suivant : ES**

1. Procédé pour stabiliser une vitamine $D_3$ active comprenant l'addition à la vitamine $D_3$ active d'un stabilisant choisi parmi le polyvinylacétal diéthylaminoacétate et l'hydroxypropylcellulose.

2. Procédé de préparation d'une vitamine $D_3$ active et stable sous une forme pour l'administration, comprenant l'addition à une vitamine $D_3$ active d'un stabilisant choisi parmi le polyvinylacétal diéthylaminoacétate et l'hydroxypropylcellulose, puis l'addition d'un support pharmaceutiquement acceptable au mélange obtenu.

3. Procédé selon la revendication 2, comprenant en outre l'addition d'un autre ingrédient pharmaceutiquement efficace.

4. Procédé selon la revendication 2, dans lequel la proportion de stabilisant est de 1 à 100.000 fois le poids de la teneur en vitamine $D_3$ active.